# FASCICULE DE BREVET EUROPEEN

(11) **EP 3 664 894 B1**
(45) Date de publication et mention de la délivrance du brevet: **29.09.2021**
(21) Numéro de dépôt: 18762232.9
(22) Date de dépôt: 10.08.2018
(51) Int. Cl.: A61K 36/064, A61P 25/24, A61P 25/22

(54) **SACCHAROMYCES BOULARDII POUR LE TRAITEMENT DES TROUBLES DE L'HUMEUR**
SACCHAROMYCES BOULARDII ZUR BEHANDLUNG VON STIMMUNGSSCHWANKUNGEN
SACCHAROMYCES BOULARDII FOR THE TREATMENT OF MOOD DISORDERS

(30) Priorité: 11.08.2017 EP 17306065
(43) Date de publication de la demande: 17.06.2020
(73) Titulaire: Biocodex, 94257 Gentilly Cedex (FR)
(72) Inventeur: SIMON O'BRIEN, Emmanuelle, 60610 Lacroix-Saint-Ouen (FR); VERLEYE, Marc, 60190 Remy (FR); LE GUERN, Marie-Emmanuelle, 60200 Compiegne (FR)
(74) Mandataire: Vial, Lionel
(86) Numéro de dépôt international: PCT/EP2018/071726
(87) Numéro de publication internationale: WO 2019/030371

(56) Documents cités:
- US-A1- 2006 140 974
- BUTS JEAN-PAUL: "Twenty-Five Years of Research on Saccharomyces boulardii Trophic Effects: Updates and Perspectives", DIGESTIVE DISEASES AND SCIENCES, vol. 54, no. 1, janvier 2009 (2009-01), pages 15-18, XP002778132,

## Description

### Domaine de l'invention

La présente invention concerne un médicament pour la prévention ou le traitement des troubles de l'humeur chez un individu, notamment pour la prévention ou le traitement des troubles dépressifs ou des troubles anxieux et de l'anxiété.

### Arrière-plan technique

Les troubles anxieux constituent un groupe de problèmes psychologiques et neurologiques se manifestant sous plusieurs formes de peur et d'anxiété anormales ou pathologiques. La cinquième édition du manuel diagnostique et statistique des troubles mentaux (DSM-5™) et la 10^{ème} révision de la classification internationale des maladies (ICD-10) de l'organisation mondiale de la santé (OMS) reconnaissent ainsi une large variété de troubles anxieux, tels que l'anxiété de séparation, le mutisme sélectif, la phobie spécifique, la phobie sociale, le trouble panique, l'agoraphobie, le trouble anxieux généralisé, le trouble anxieux induit par une substance/un médicament, et le trouble anxieux induit par une autre affection médicale.

Si les principaux axes de lutte contre ces troubles restent la thérapie comportementale et les médicaments de type anxiolytique ou antidépresseur, notamment les benzodiazépines, telles que le diazépam (Valium®) ou l'alprazolam (Xanax®), qui constituent actuellement le traitement pharmacologique de référence, d'autres approches sont également explorées afin de limiter les effets secondaires de ces derniers composés.

Ainsi, Desbonnet et al. (2010) Neuroscience 170:1179 -1188 ont par exemple montré que *Bifidobacterium infantis* 35624 permettait de diminuer les comportements dépressifs mesurés par le test de nage forcée dans le modèle de dépression induite par la séparation maternelle chez le rat. Toutefois, l'efficacité du probiotique est limitée par rapport à l'antidépresseur utilisé dans cette étude, à savoir le citalopram.

Il reste donc nécessaire d'identifier d'autres alternatives aux anxiolytiques actuels ayant une efficacité comparable à ces derniers.

*Saccharomyces boulardii,* également nommée Saccharomyces cerevisiae var. *boulardii,* est une souche particulière de la levure Saccharomyces cerevisiae. Ce probiotique est principalement indiqué en complément de la réhydratation pour le traitement des diarrhées. Son utilité a été notamment établie chez l'enfant (Villarruel et al. (2007) Acta Paediatr 96:538-541 ; Szajewska et al. (2007) Aliment Pharmacol Ther 25:257-264) et pour les diarrhées liées à la prise d'antibiotiques (Surawicz et al. (1989) Gastroenterology 96:981-988; Kotowska et al. (2005) Aliment Pharmacol Ther 21:583-590) ou aux infections à *Clostridium difficile* (Surawicz et al. (2000) Clin Infect Dis 31:1012-1017).

### Résumé de l'invention

La présente invention découle de la mise en évidence inattendue, par les inventeurs, que l'administration de cellules de levure *Saccharomyces boulardii* à des souris permettait de diminuer l'anxiété chez ces souris.

Ainsi, la présente invention concerne des cellules de levure *Saccharomyces boulardii* pour une utilisation :
- dans la prévention ou le traitement des troubles de l'humeur chez un individu, et/ou
- dans la prévention ou le traitement des troubles dépressifs ou de la dépression, ou pour une utilisation à titre d'antidépresseur, chez un individu, et/ou
- dans la prévention ou le traitementou des troubles anxieux ou de l'anxiété, ou pour une utilisation à titre d'anxiolytique, chez un individu.

Dans un mode de réalisation particulier de l'invention, les cellules de levure *Saccharomyces boulardii* pour une utilisation telle que définie ci-dessus sont en combinaison avec au moins une autre substance destinée à la prévention ou au traitement des troubles de l'humeur chez un individu, notamment à la prévention ou au traitement des troubles dépressifs ou de la dépression, ou des troubles anxieux ou de l'anxiété.

La présente invention concerne également une composition pharmaceutique ou médicament comprenant des cellules de levure *Saccharomyces boulardii* à titre de principe actif et éventuellement au moins un véhicule ou excipient pharmaceutiquement acceptable, pour une utilisation dans la prévention ou le traitement des troubles de l'humeur chez un individu, notamment dans la prévention ou le traitement des troubles dépressifs ou de la dépression, ou des troubles anxieux ou de l'anxiété, ou pour une utilisation à titre d'antidépresseur ou d'anxiolytique.

Dans un mode de réalisation particulier de l'invention, la composition pharmaceutique ou le médicament pour une utilisation telle que définie ci-dessus, comprend au moins une autre substance destinée à la prévention ou au traitement des troubles de l'humeur chez un individu, notamment à la prévention ou au traitement des troubles dépressifs ou de la dépression, ou des troubles anxieux ou de l'anxiété.

La présente invention concerne également une méthode de prévention ou de traitement d'un trouble de l'humeur chez un individu, notamment une méthode de prévention ou de traitement d'un trouble dépressif ou de la dépression, ou d'un trouble anxieux ou de l'anxiété chez un individu, comprenant l'administration à l'individu d'une quantité efficace de cellules de levure *Saccharomyces boulardii.*

Dans un mode de réalisation particulier la méthode selon l'invention, les cellules de levure sont en combinaison avec au moins une autre substance destinée à la prévention ou au traitement des troubles de l'humeur chez un individu, notamment à la prévention ou au traitement des troubles dépressifs ou de la dépression, ou des troubles anxieux ou de l'anxiété.

La présente invention concerne également l'utilisation de cellules de levure *Saccharomyces boulardii* pour la préparation d'un médicament destiné à la prévention ou au traitement des troubles de l'humeur chez un individu, notamment à la prévention ou au traitement des troubles dépressifs ou de la dépression, ou de troubles anxieux ou de l'anxiété.

Dans un mode de réalisation particulier de l'utilisation telle que définie ci-dessus l'invention, le médicament comprend au moins une autre substance destinée à la prévention ou au traitement des troubles de l'humeur chez un individu, notamment à la prévention ou le traitement des troubles dépressifs ou de la dépression, ou des troubles anxieux ou de l'anxiété.

La présente invention concerne également une composition pharmaceutique ou un médicament comprenant à titre de substance active :
- des cellules de levure *Saccharomyces boulardii,* et
- au moins une autre substance destinée à la prévention ou au traitement des troubles de l'humeur chez un individu, notamment à la prévention ou au traitement des troubles dépressifs ou de la dépression, ou des troubles anxieux ou de l'anxiété, éventuellement en association avec au moins un véhicule ou excipient pharmaceutiquement acceptable.

La présente invention concerne également des produits contenant :
- des cellules de levure *Saccharomyces boulardii,* et
- au moins une autre substance destinée à la prévention ou au traitement des troubles de l'humeur chez un individu, notamment à la prévention ou ou traitement des troubles dépressifs ou de la dépression, ou des troubles anxieux ou de l'anxiété, comme produit de combinaison pour une utilisation séparée, simultanée ou étalée dans le temps pour la prévention ou le traitement des troubles de l'humeur chez un individu, notamment pour la prévention ou le traitement des troubles dépressifs ou de la dépression, ou des troubles anxieux ou de l'anxiété, ou pour une utilisation à titre d'antidépresseur ou d'anxiolytique.

### Description détaillée de l'invention

A titre préliminaire, on rappellera que le terme «comprenant» signifie «incluant», «contenant» ou «englobant», c'est-à-dire que lorsqu'un objet «comprend» un élément ou plusieurs éléments, d'autres éléments que ceux mentionnés peuvent également être compris dans l'objet. A contrario, l'expression «consistant en» signifie «constitué de», c'est-à-dire que lorsqu'un objet «consiste en» un élément ou plusieurs éléments, l'objet ne peut pas comprendre d'autres éléments que ceux mentionnés.

Par ailleurs, comme l'homme du métier le comprendra bien, l'expression «Substance ou composition pour une utilisation dans la prévention ou le traitement d'une maladie » est synonyme de l'expression « Substance ou composition pour une utilisation dans une méthode de prévention ou de traitement d'une maladie ».

### Troubles de l'humeur

Comme on l'entend ici «la prévention ou le traitement» des troubles de l'humeur, notamment des troubles dépressifs ou de la dépression, ou des troubles anxieux ou de l'anxiété, vise à traiter thérapeutiquement, à atténuer, ou à prévenir, de manière prophylactique, les troubles de l'humeur, notamment les troubles dépressifs ou la dépression, ou les troubles anxieux ou l'anxiété.

Les «troubles de l'humeur» sont bien connus de l'homme du métier et comprennent notamment les troubles dépressifs ou la dépression et les troubles anxieux ou l'anxiété. Les « troubles dépressifs» sont bien connus de l'homme du métier et sont notamment définis dans le chapitre «Troubles dépressifs» (« Depressive Disorders») pages 155 à 188 de la cinquième édition du manuel diagnostique et statistique des troubles mentaux («Diagnostic and statistical manual of mental disorders», 5ème édition, DSM-5™, 2013, American Psychiatric Association) et correspondent notamment aux classes F20.4, F25.1, F31.3, F31.4, F31.5, F32, F33, F41.2, F92.0 de la dixième révision de la classification internationale des maladies version 2016 (ICD-10:2016).

De préférence, les troubles dépressifs selon l'invention sont le trouble dépressif induit par une substance/un médiament, le trouble disruptif avec dysrégulation de l'humeur, la dépression majeure, le trouble dépressif persistant, le trouble dépressif induit par une autre affection médicale, ainsi que plus généralement tout trouble dépressif spécifié et non spécifié.

Les « troubles anxieux» et « l'anxiété » sont bien connus de l'homme du métier et sont notamment définis dans le chapitre «Troubles anxieux» («Anxiety Disorders») pages 189 à 233 de la cinquième édition du manuel diagnostique et statistique des troubles mentaux («Diagnostic and statistical manual of mental disorders», 5ème édition, DSM-5™, 2013, American Psychiatric Association) et correspondent notamment aux classes F06.4, F40, F41, F93 et F94 de la dixième révision de la classification internationale des maladies version 2016 (ICD-10:2016).

De préférence, les troubles anxieux ou l'anxiété selon l'invention sont l'anxiété de séparation, le mutisme sélectif, la phobie spécifique, la phobie sociale, le trouble panique, l'agoraphobie, le trouble anxieux généralisé, le trouble anxieux induit par une substance/un médicament, et le trouble anxieux induit par une autre affection médicale, ainsi que plus généralement tout trouble anxieux spécifié et non spécifié.

### Cellules de levure

*Saccharomyces boulardii,* abréviée *S. boulardii,* est une levure bien connue de l'homme du métier. Elle est notamment décrite dans Hennequin et al. (2001) J. Clin. Microbiol. 39:551-559. Comme on l'entend ici, les nomenclatures "Saccharomyces *boulardii"* et "*Saccharomyces cerevisiae var. boulardii"* (abréviée *S. cerevisiae var*. *boulardii*) sont considérées équivalentes.

Comme on l'entend ici, l'expression « cellules de levure » regroupe des cellules de levure viables ou mortes, entières ou sous forme de débris. De préférence, au moins une partie des cellules de levure *Saccharomyces boulardii* selon l'invention sont viables, notamment viables et cultivables, et plus préférablement une majorité des cellules de levures *Saccharomyces boulardii* selon l'invention sont viables, notamment viables et cultivables.

La viabilité d'une cellule de levure peut notamment être déterminée par coloration au bleu de méthylène et observation microscopique. Le nombre de cellules viables et cultivables, ce qui définit la vitalité, peut être estimé en déterminant le nombre d'Unités Formant Colonie (UFC) comprises dans l'échantillon.

A titre d'exemple, le nombre d'UFC de cellules de levure d'un échantillon liquide comprenant des levures peut être déterminé en étalant un volume déterminé de l'échantillon sur un milieu solide, par exemple gélosé, permettant la croissance des levures et en incubant le milieu solide pendant une durée, par exemple 48 h, et à une température, par exemple 30°C, permettant la croissance de colonies de levures. Le nombre de colonies rapporté au volume étalé sur le milieu solide permet de déterminer le nombre d'UFC compris dans l'échantillon. Un protocole détaillé de la détermination d'UFC conforme à l'invention est notamment décrit dans Toothaker & Elmer (1984) Antimicrobial Agents and Chemotherapy 26:552-556 au paragraphe « Assay for S. boulardii». Par ailleurs, lorsque l'échantillon de levure se présente sous forme d'un solide, par exemple une poudre lyophilisée, on préfère déterminer le nombre d'UFC compris dans l'échantillon après avoir repris une masse déterminée de l'échantillon dans une solution aqueuse, notamment de l'eau distillée ou une solution à 0,9% de NaCl à pH 7.

Une « levure » selon l'invention est un champignon de préférence unicellulaire. Les cellules de levure selon l'invention sont de l'espèce *Saccharomyces boulardii. Saccharomyces boulardii* est bien connu de l'homme du métier et est notamment décrit dans Hennequin et al. (2001) J. Clin. Microbiol. 39:551-559.

De manière particulièrement préférée, les cellules de levure Saccharomyces *boulardii* selon l'invention sont obtenues à partir de médicaments de marque Ultra-Levure®, Bioflor®, Codex®, Econorm®, Enflor®, Enterol®, Florastor®, Floratil®, Florestor®, Inteflora®, Perenterol®, Perenteryl®, Precosa®, Reflor®, Ultra-Levura®, ou à partir des dépôts effectués à l'American Type Culture Collection (ATCC, USA) sous la référence 74012 ou à la Collection Nationale de Culture et de Microorganismes (CNCM, France) sous la référence I-745.

De préférence, également les cellules de levure *Saccharomyces boulardii* selon l'invention sont lyophilisées, telles que les cellules de levure Saccharomyces boulardii de marque Ultra-Levure®, Bioflor®, Codex®, Econorm®, Enflor®, Enterol®, Florastor®, Floratil®, Florestor®, Inteflora®, Perenterol®, Perenteryl®, Precosa®, Reflor®, ou Ultra-Levura®.

De manière avantageuse, la viabilité et la vitalité de cellules de levure obtenues à partir de lyophilisats sont supérieures à ce qui peut être obtenu à partir d'autres modes de conservation des cellules de levure.

Comme on l'entend ici, la « lyophilisation » est une méthode de conservation dans laquelle les cellules de levure sont congelées puis soumises à une sublimation de l'eau congelée qu'elles contiennent pour donner un lyophilisat sous forme d'une poudre de levure sèche contenant de préférence moins de 2% d'eau et plus préférablement moins de 1% d'eau. De préférence, les cellules de levure lyophilisées sont obtenues à partir de concentrés de cellules de levure. N'importe quel type de méthode de lyophilisation de cellules de levure connu de l'homme du métier peut être utilisé. Toutefois, les cellules de levure sont de préférence lyophilisées selon l'invention à l'aide de la méthode de lyophilisation suivante :
- cultiver les cellules de levure dans un milieu nutritif liquide jusqu'à ce que les cellules atteignent une phase stationnaire ;
- concentrer les cellules de levure cultivées et congeler le concentré ;
- lyophiliser le concentré.

Les cellules de levure *Saccharomyces boulardii* lyophilisées sont sous forme d'une poudre.

### Individu

L'individu selon l'invention est un animal, de préférence un mammifère, et plus préférablement un humain. Comme cela sera clair pour l'homme du métier, l'individu selon l'invention présente un trouble de l'humeur selon l'invention, notamment un trouble dépressif ou une dépression selon l'invention ou un trouble anxieux ou de l'anxiété selon l'invention,ou est à risque de présenter un trouble de l'humeur selon l'invention, notamment un trouble dépressif ou une dépression selon l'invention ou un trouble anxieux ou de l'anxiété selon l'invention, autrement dit l'individu selon l'invention a besoin d'un traitement antidépresseur ou anxiolytique.

### Autre substance

Comme on l'entend ici, l'expression « autre substance destinée à la prévention ou au traitement d'un trouble de l'humeur, notamment à la prévention ou au traitement d'un trouble dépressif ou de la dépression, ou d'un trouble anxieux ou de l'anxiété » concerne notamment tout composé ou probiotique, différent des cellules de levure *Saccharomyces boulardii* destiné à traiter, atténuer ou prévenir les troubles de l'humeur, notamment les troubles dépressifs ou la dépression, ou les troubles anxieux ou l'anxiété. La substance est ainsi préférentiellement une substance antidépressive, également nommée «antidépresseur», ou une substance anxiolytique, également nommée « anxiolytique ».

Comme on l'entend ici le terme «antidépresseur» ou «antidépressive» concerne notamment tout composé ou probiotique, différent des cellules de levure *Saccharomyces boulardii* destiné à traiter, atténuer ou prévenir les troubles dépressifs.

Comme on l'entend ici le term «anxiolytique» concerne notamment tout composé ou probiotique, différent des cellules de levure *Saccharomyces boulardii* destiné à traiter, atténuer ou prévenir les troubles anxieux ou de l'anxiété.

Comme on l'entend ici, le terme « probiotique » désigne tout microorganisme, de préférence viable, administré à un individu, notamment en vue d'améliorer son état de santé.

De préférence, l'autre substance destinée à la prévention ou au traitement d'un trouble de l'humeur, notamment à la prévention ou au traitement d'un trouble dépressif ou de la dépression, ou d'un trouble anxieux ou de l'anxiété, est sélectionnée dans le groupe constitué :
- d'une benzodiazépine, telle que le bromazépam, le clobazam, le prazépam, de lorazépam, le clorazépate dipotassique, le clotiazépam, le Ioflazépate d'éthyle, l'oxazépam, le diazépam, l'alprazolam, ou le clonazépam ;
- d'un inhibiteur sélectif de recapture de la sérotonine, tel que le citalopram l'escitalopram, la fluoxétine, la fluvoxamine, la sertraline, ou la paroxétine ;
- d'un inhibiteur de monoamine oxydase (MOA), tel que le moclobémide ou l'iproniazide ;
- d'un inhibiteur de la recapture de la sérotonine et des catécholamines, en particulier de la noradrénaline, tel que la duloxétine, la venlafaxine, le milnacipran, l'imipramine, amitriptyline, la clomipramine, la dosulépine, la doxépine, la mirtazapine, ou la miansérine ; ou
- de l'etifoxine, de l'hydroxyzine, de la buspirone, de la tianeptine, de l'aglomélatine, ou du captodiame.

Par ailleurs, dans un mode de réalisation particulier de l'invention, les cellules de levures *Saccharomyces boulardii* selon l'invention ne sont pas administrées avec une autre substance anxiolytique ou antidépressive, ou destinée à la prévention ou au traitement d'un trouble de l'humeur, notamment à la prévention ou au traitement d'un trouble dépressif ou de la dépression, ou d'un trouble anxieux ou de l'anxiété, et la composition pharmaceutique, le médicament ou les produits selon l'invention ne comprenent pas d'autre substance anxiolytique ou antidépressive, ou destinée à la prévention ou au traitement d'un trouble de l'humeur, notamment à la prévention ou au traitement d'un trouble dépressif ou de la dépression, ou d'un trouble anxieux ou de l'anxiété, que les cellules de levure *Saccharomyces boulardii* selon l'invention.

Dans un autre mode de réalisation particulier de l'invention, les cellules de levure *Saccharomyces boulardii* ne sont pas administrées avec un autre probiotique, et la composition pharmaceutique, le médicament ou les produits selon l'invention ne comprennent pas de probiotique autre que *Saccharomyces boulardii.*

Dans encore un autre mode de réalisation particulier de l'invention, les cellules de levure *Saccharomyces boulardii* ne sont pas administrées avec l'acide orotique ou un sel de celui-ci, ou avec *Bifidobacterium longum,* notamment *Bifidobacterium longum* ATCC BAA-999, et la composition pharmaceutique, le médicament ou les produits selon l'invention ne comprennent pas l'acide orotique ou un sel de celui-ci, ou *Bifidobacterium longum,* notamment *Bifidobacterium longum* ATCC BAA-999.

### Administration

De préférence, les cellules de levure *Saccharomyces boulardii,* la composition pharmaceutique, le médicament et les produits selon l'invention sont sous une forme adaptée à une administration par voie orale. Ainsi, les cellules de levure *Saccharomyces boulardii,* la composition pharmaceutique, le médicament et les produits selon l'invention sont, ou se présentent, de préférence sous forme de gélules ou de sachets de poudre.

De préférence également, les cellules de levure *Saccharomyces boulardii* selon l'invention sont administrées à une dose de 0,5.10⁸ à 100.10¹⁰ UFC/kg/j ou à une dose de 0,00125 g/kg/j à 25 g/kg/j.

De préférence également, les cellules de levure *Saccharomyces boulardii* selon l'invention sont administrées à une dose de 0,5 g/kg à 10 g/kg, plus préférablament à une dose de 1 à 6 g/kg et encore plus préférablement à une dose d'environ 3 g/kg, en particulier 1, 2, 3, 4 ou 5 fois par jour.

Comme l'homme du métier le comprendra, la quantité de cellules de levure à administrer par unité de masse (kg) se réfère à la masse de l'individu auquel sont destinées les cellules de levure. Par ailleurs, lorsque la quantité de cellules de levure à administrer est exprimée en unité de masse (g), les cellules de levure sont de préférence sous forme lyophilisée.

De préférence, la composition pharmaceutique, le médicament ou les produits selon l'invention, comprenannt les cellules de levure *Saccharomyces boulardii* en une dose de 50 mg à 250 mg. En outre, les cellules de levure *Saccharomyces boulardii* pour leur utilisation telle que définie ci-dessus sont de préférence administrée à une dose unitaire de 50 mg à 250 mg.

Comme on l'entend ici l'expression « en combinaison » ou « produit de combinaison » signifie que (i) les cellules de levure *Saccharomyces boulardii* selon l'invention et (ii) l'autre substance destinée à la prévention ou au traitement d'un trouble de l'humeur, notamment à la prévention ou au traitement d'un trouble dépressif ou de la dépression, ou d'un trouble anxieux ou de l'anxiété selon l'invention peuvent être associées au sein d'une même composition pharmaceutique ou d'un même médicament, et donc être administrées ensemble, ou bien être administrés de manière séparée, c'est-à-dire selon des voies d'administration distinctes et/ou des régimes d'administration distincts, sous réserve que lorsqu'elles sont administrées de manière séparée les périodes d'activité anti-troubles de l'humeur respectives des cellules de levure *Saccharomyces boulardii* et de l'autre substance se recouvrent en totalité ou en partie, notamment de manière qu'elles puissent coopérer pour exercer un effet anti-troubles de l'humeur synergique.

L'invention sera davantage explicitée de manière non limitative à l'aide des figues et des Exemples qui suivent.

### Description des figures

Figure 1

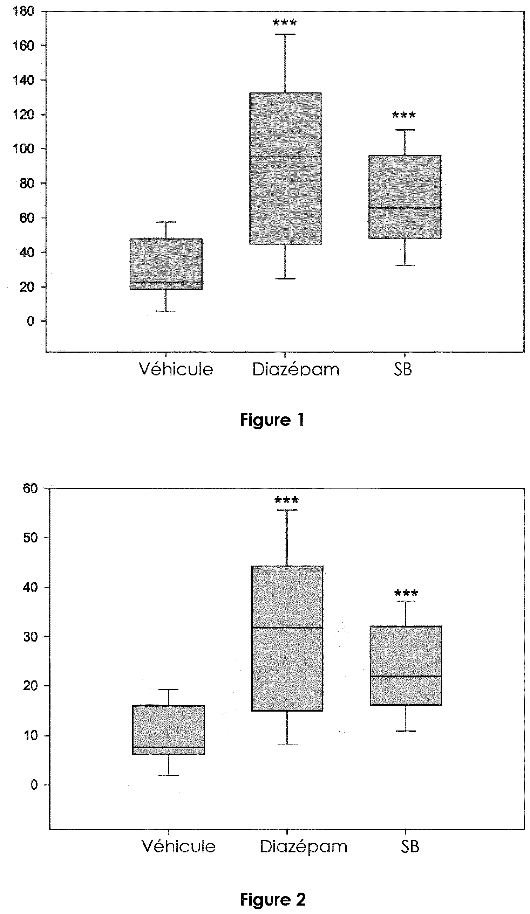

   La figure 1 représente un diagramme en boîte du temps passé (en secondes) dans les quadrants ouverts (axe vertical) par les souris témoins (véhicule, = 20), par les souris traitées au diazépam (diazépam, n = 10) ainsi que par les souris traitées par des cellules de levure *Saccharomyces boulardii* (SB, n = 10). Les souris traitées par SB (3 g/kg, p.o.) pendant 12 jours ou avec une administration unique de diazépam (1 mg/kg, i.p.) passent significativement plus de temps dans les quadrants ouverts que les souris témoins. Une ANOVA à un facteur de Kruskal-Wallis a été réalisée suivie d'un test de Dunn. Le symbole trois étoiles (***) représente p < 0,05.
Figure 2
   La figure 2 représente un diagramme en boîte du pourcentage de temps passé dans les quadrants ouverts (axe vertical) par les souris témoins (véhicule, n = 20), par les souris traitées au diazépam (diazépam, n = 10) ainsi que par les souris traitées par les cellules de levure *Saccharomyces boulardii* (SB, n = 10). Les souris traitées par SB (3 g/kg, p.o.) pendant 12 jours ou avec une administration unique de diazépam (1 mg/kg, i.p.) passent significativement plus de temps dans les quadrants ouverts que les souris témoins. Une ANOVA à un facteur de Kruskal-Wallis a été réalisée suivie d'un test de Dunn. Le symbole trois étoiles (***) représente p< 0,05.

### EXEMPLES

### EXEMPLE 1

Les inventeurs ont évalué les effets d'un traitement chronique avec Saccharomyces *boulardii* (SB) sur le comportement anxieux basal de la souris CD1 dans le modèle animal d'anxiété dit du labyrinthe en zéro surélevé (*elevated zéro* maze) initialement décrit par Shepherd et al. (1994) Psychopharmacology (Berl). 116:56-64 puis repris par d'autres (voir par exemple Kulkarni et al. (2007) Methods Find Exp Clin Pharmacol. 29: 343-348 et Braun et al. (2011) Pharmacol. Biochem. Behav. 97:406-415).

### 1. Matériel et méthodes

### 1.1. Animaux

Des souris mâles adultes CD1 (élevage Charles River) âgées de 7 semaines et de poids compris entre 27 et 37 grammes au moment de leur arrivée sont utilisées après une acclimatation d'au moins 1 semaine dans l'animalerie. Les conditions d'hébergement sont les suivantes : t° ambiante = 22 ± 2°C ; nourriture Safe « A04 », eau du robinet comme eau de boisson, cycle nycthéméral : 12 h/12 h (lumière : 7 h/19 h - obscurité : 19 h/7 h). Les souris sont hébergées à 10 par cage dans des cages de type 1500U. Les expériences sont réalisées selon les recommandations européennes (directive 2010/63/UE) pour l'usage des animaux de laboratoire et ont été validées et approuvées par le comité d'éthique (projet numéro 2200.01).

### 1.2. Traitement

Un groupe de souris (n = 10) a reçu des cellules de levure *Saccharomyces boulardii* (SB) CNCM I-745 lyophilisées (lot 9289 avec une vitalité de 31 x 10⁹CFU/g) solubilisées dans de l'eau du robinet, par voie orale à la dose de 3 g/kg et avec un volume de 10 ml/kg, 2 fois par jour pendant 12 jours (de 9h30 à 10h30 et de 15h30 à 16h30). Un second groupe de souris (n = 10) a reçu du diazépam (Valium®, Roche, lot F1081F01), anxiolytique de référence, à la dose de 1 mg/kg par voie intrapéritonéale (i.p.) avec un volume de 10 mL/kg, 30 minutes avant le test comportemental. Le groupe témoin (n = 10) correspondant au traitement SB a reçu le liquide véhicule seul (eau) à la même fréquence et avec la même voie d'administration que les animaux traités. Le groupe témoin (n = 10) correspondant au traitement diazépam a reçu le liquide véhicule (NaCl 0.9%) à la même fréquence et avec la même voie d'administration que les animaux traités. Les résultats des 2 groupes témoins n'étant pas significativement différents, leurs résultats ont été regroupés afin de clarifier les graphiques.

### 1.3. Protocole

Le labyrinthe en zéro surélevé est constitué d'une plateforme ronde (2 quadrants ouverts et 2 quadrants fermés) de 50 cm de diamètre et de 5 cm de large située à 40 cm du sol. Une paroi opaque de 15 cm de haut délimite les quadrants fermés et un rebord de 3 mm entoure les zones ouvertes. La luminosité était de 30 lux dans les zones ouvertes et de 3 lux dans les zones fermées. Les déplacements des animaux sont détectés grâce à un plancher infrarouge et analysés par logiciel VideoTrack V2.5 (ViewPoint). Après une habituation d'au moins 45 minutes, le test commence lorsque l'animal est posé au centre d'un quadrant fermé, et dure 5 minutes.

L'entrée dans un nouveau quadrant est considérée lorsque l'animal pose les quatre pattes dans la zone. Les paramètres mesurés sont : le temps passé et le pourcentage de temps passé dans les quadrants ouverts. Un niveau d'anxiété plus élevé est normalement associé à une quantité inférieure de temps passé dans les quadrants ouverts (zones plus anxiogènes) synonyme d'une activité exploratoire réduite.

### 1.4. Analyse statistique

Les résultats sont exprimés par la moyenne ± ESM. Les résultats sont analysés à l'aide d'une analyse de variance (ANOVA) de Kruskal-Wallis à un facteur [traitement]. En cas de résultat statistiquement significatif, un test de comparaison multiple (Dunn) est effectué entre les groupes pour déterminer ceux qui diffèrent au seuil de 5% (SigmaStat, V3.5, Systat Software Inc.).

### 2. Résultats

Les résultats de l'expérience montrent que les souris traitées avec les cellules de la levure *Saccharomyces boulardii* (SB) 2 fois par jour pendant 12 jours passent significativement plus de temps dans les quadrants ouverts que les souris témoins (71,6 sec pour les souris traitées par SB contre 31,2 sec pour les souris témoins, ou 23,9 % contre 10,4 % du temps) (p < 0,05). Les souris traitées au diazépam, anxiolytique de référence, passent également significativement plus de temps dans les quadrants ouverts en comparaison avec les témoins (91,9 sec ou 30,6 % du temps pour les souris traitées au diazépam) (p < 0,05) (**Figures 1 et 2**).

### Conclusion

Les résultats de cette étude montrent qu'un traitement chronique de 2 semaines avec *Saccharomyces boulardii* chez la souris mâle CD1 augmente significativement le temps passé dans les quadrants ouverts du labyrinthe en zéro surélevé, au même titre qu'une administration de diazépam. Ces résultats démontrent ainsi un effet bénéfique de la levure *Saccharomyces boulardii* sur l'anxiété.

### EXEMPLE 2

Les effets antidépresseurs de la levure *Saccharomyces boulardii* sont évalués par administration chronique chez la souris mâle adulte CD1 dans le test de la nage forcée.

Le test de la nage forcée, bien connu de l'homme du métier, permet de mesurer les effets antidépresseurs d'un composé pharmacologique. Ce test se base sur les travaux de Porsolt et al. (1977) Act. Int. Pharmacodyn. Ther. 229:327-336 et est classiquement utilisé depuis pour prédire l'efficacité clinique de composés antidépresseurs.

Brièvement, ce test se déroule dans un récipient cylindrique rempli d'eau (hauteur d'eau 10cm) à 23°C. La souris est placée dans ce récipient pendant 6 minutes, et la durée de l'immobilité de l'animal est mesurée pendant les 4 dernières minutes.

Les composés antidépresseurs administrés en amont de ce test diminuent significativement le temps d'immobilité des animaux.

## Revendications

1. Cellules de levure *Saccharomyces boulardii* pour une utilisation dans la prévention ou le traitement des troubles de l'humeur chez un individu.

2. Cellules de levure *Saccharomyces boulardii* pour une utilisation selon la revendication 1, dans la prévention ou le traitement des troubles dépressifs, notamment sélectionnés dans le groupe constitué du trouble dépressif induit par une substance/un médicament, du trouble disruptif avec dysrégulation de l'humeur, de la dépression majeure, du trouble dépressif persistant, du trouble dépressif induit par une autre affection médicale, et tout trouble dépressif spécifié et non spécifié, ou de la dépression, ou pour une utilisation à titre d'antidépresseur, chez un individu.

3. Cellules de levure *Saccharomyces boulardii* pour une utilisation selon la revendication 1 ou 2, dans la prévention ou le traitement des troubles anxieux ou de l'anxiété, notamment sélectionnés dans le groupe constitué de l'anxiété de séparation, le mutisme sélectif, la phobie spécifique, la phobie sociale, le trouble panique, l'agoraphobie, le trouble anxieux généralisé, le trouble anxieux induit par une substance/un médicament, le trouble anxieux induit par une autre affection médicale, et tout trouble anxieux spécifié et non spécifié, ou pour une utilisation à titre d'anxiolytique, chez un individu.

4. Cellules de levure *Saccharomyces boulardii* pour une utilisation selon l'une des revendications 1 à 3, les cellules de levure *Saccharomyces boulardii* étant lyophilisées.

5. Cellules de levure de *Saccharomyces boulardii* pour une utilisation selon l'une des revendications 1 à 4, les cellules de levure *Saccharomyces boulardii* étant sous une forme adaptée à une administration par voie orale, notamment sous forme de gélules ou de sachets de poudre.

6. Cellules de levure *Saccharomyces boulardii* pour une utilisation selon l'une des revendications 1 à 5, les cellules de levure étant administrées à une dose de 0,00125 à 25 g/kg/j.

7. Cellules de levure *Saccharomyces boulardii* pour une utilisation selon l'une des revendications 1 à 6, en combinaison avec au moins une autre substance destinée à la prévention ou au traitement des troubles de l'humeur.

8. Composition pharmaceutique ou médicament comprenant des cellules de levure *Saccharomyces boulardii* à titre de principe actif et éventuellement au moins un véhicule ou excipient pharmaceutiquement acceptable, pour une utilisation dans la prévention ou le traitement des troubles de l'humeur, en particulier des troubles dépressifs ou de la dépression, notamment sélectionnés dans le groupe constitué du trouble dépressif induit par une substance/un médicament, du trouble disruptif avec dysrégulation de l'humeur, de la dépression majeure, du trouble dépressif persistant, du trouble dépressif induit par une autre affection médicale, et tout trouble dépressif spécifié et non spécifié, ou des troubles anxieux ou de l'anxiété, notamment sélectionnés dans le groupe constitué de l'anxiété de séparation, le mutisme sélectif, la phobie spécifique, la phobie sociale, le trouble panique, l'agoraphobie, le trouble anxieux généralisé, le trouble anxieux induit par une substance/un médicament, et le trouble anxieux induit par une autre affection médicale.

9. Composition pharmaceutique ou médicament pour une utilisation selon la revendication 8, où les cellules de levure *Saccharomyces boulardii* sont lyophilisées.

10. Composition pharmaceutique ou médicament pour une utilisation selon la revendication 8 ou 9, se présentant sous une forme adaptée à une administration par voie orale, notamment sous forme de gélules ou de sachets de poudre.

11. Composition pharmaceutique ou médicament pour une utilisation selon l'une des revendications 8 à 10, comprenant les cellules de levure *Saccharomyces boulardii* en une dose unitaire de 50 à 250 mg.

12. Composition pharmaceutique ou médicament pour une utilisation selon l'une des revendications 8 à 11, comprenant au moins une autre substance destinée à la prévention ou au traitement des troubles de l'humeur.

13. Composition pharmaceutique ou médicament comprenant à titre de substance active :
- des cellules de levure *Saccharomyces boulardii,* et
- au moins une autre substance destinée à la prévention ou au traitement des troubles de l'humeur,
éventuellement en association avec au moins un véhicule ou excipient pharmaceutiquement acceptable.

14. Produits contenant :
- des cellules de levure *Saccharomyces boulardii,* et
- au moins une autre substance destinée à la prévention ou au traitement des troubles de l'humeur,
comme produit de combinaison pour une utilisation séparée, simultanée ou étalée dans le temps pour la prévention ou le traitement des troubles de l'humeur, notamment dans la prévention ou le traitement des troubles dépressifs ou de la dépression, ou des troubles anxieux ou de l'anxiété, ou pour une utilisation à titre d'antidépresseur ou d'anxiolytique.

## Patentansprüche

1. Zellen der Hefe *Saccharomyces boulardii* zum Gebrauch in der Vorbeugung oder Behandlung affektiver Störungen eines Patienten.

2. Zellen der Hefe *Saccharomyces boulardii* zum Gebrauch nach Patentanspruch 1 in der Vorbeugung oder Behandlung depressiver Störungen, insbesondere gewählt aus der Gruppe, bestehend aus durch eine Substanz/ein Medikament verursachte depressive Störung, disruptive Störung mit Launenfehlregulation, schwere Depression, Dythymie, durch eine andere Gesundheitsstörung hervorgerufene Depression, und jegliche spezifizierte und nicht spezifizierte depressive Störung, oder von Depressionen, oder zum Gebrauch als Antidepressivum bei einem Patienten.

3. Zellen der Hefe *Saccharomyces boulardii* zum Gebrauch nach Patentanspruch 1 oder 2, in der Vorbeugung oder Behandlung von Angststörungen oder Angst, insbesondere gewählt aus der Gruppe, bestehend aus Trennungsangst, selektivem Mutismus, spezifischer Phobie, sozialer Phobie, Panikstörung, Agoraphobie, generalisierter Angststörung, durch eine Substanz/ein Medikament verursachte Angststörung, durch eine andere Gesundheitsstörung hervorgerufene Angststörung, und jegliche spezifizierte oder nicht spezifizierte Angststörung, oder zum Gebrauch als Anxiolytikum bei einem Patienten.

4. Zellen der Hefe *Saccharomyces boulardii* zum Gebrauch nach irgendeinem der Patentansprüche 1 bis 3, wobei die Zellen der Hefe *Saccharomyces boulardii* lyophilisiert sind.

5. Zellen der Hefe *Saccharomyces boulardii* zum Gebrauch nach irgendeinem der Patentansprüche 1 bis 4, wobei die Zellen der Hefe *Saccharomyces boulardii* in einer Form vorliegen, die zur oralen Verabreichung geeignet ist, insbesondere als Gelkapseln oder Pulvertütchen.

6. Zellen der Hefe *Saccharomyces boulardii* zum Gebrauch nach irgendeinem der Patentansprüche 1 bis 5, wobei die Hefezellen in einer Dosierung von 0,00125 bis 25 g/kg/d verabreicht werden.

7. Zellen der Hefe *Saccharomyces boulardii* zum Gebrauch nach irgendeinem der Patentansprüche 1 bis 6 in Verbindung mit mindestens einer anderen Substanz, die zur Vorbeugung oder Behandlung affektiver Störungen bestimmt ist.

8. Pharmazeutische Zubereitung oder Medikament, als Wirkstoff Zellen der Hefe *Saccharomyces boulardii* enthaltend und gegebenenfalls mindestens einen pharmazeutisch verträglichen Träger oder Vehikel, zum Gebrauch in der Vorbeugung oder Behandlung affektiver Störungen, insbesondere depressiver Störungen oder von Depression, insbesondere gewählt aus der Gruppe, bestehend aus durch eine Substanz/ein Medikament verursachte depressive Störung, disruptive Störung mit Launenfehlregulation, schwere Depression, Dythymie, durch eine andere Gesundheitsstörung hervorgerufene Depression, und jegliche spezifizierte oder nicht spezifizierte depressive Störung, oder von Angststörungen oder Angst, insbesondere gewählt aus der Gruppe, bestehend aus Trennungsangst, selektivem Mutismus, spezifischer Phobie, sozialer Phobie, Panikstörung, Agoraphobie, generalisierter Angststörung, durch eine Substanz/ein Medikament hervorgerufene Angststörung, und durch eine andere Gesundheitsstörung hervorgerufene Angststörung.

9. Pharmazeutische Zubereitung oder Medikament zum Gebrauch nach Patentanspruch 8, wobei die Zellen der Hefe *Saccharomyces boulardii* lyophilisiert sind.

10. Pharmazeutische Zubereitung oder Medikament zum Gebrauch nach irgendeinem der Patentansprüche 8 oder 9 in einer Form, die zur oralen Verabreichung geeignet ist, insbesondere als Gelkapseln oder Pulvertütchen.

11. Pharmazeutische Zubereitung oder Medikament zum Gebrauch nach irgendeinem der Patentansprüche 8 bis 10, die Zellen der Hefe *Saccharomyces boulardii* in einer Einzeldosis von 50 bis 250 mg enthaltend.

12. Pharmazeutische Zubereitung oder Medikament zum Gebrauch nach irgendeinem der Patentansprüche 8 bis 11, mindestens eine andere Substanz, die zur Vorbeugung oder Behandlung affektiver Störungen bestimmt ist, enthaltend.

13. Pharmazeutische Zubereitung oder Medikament, als Wirkstoff enthaltend:
- Zellen der Hefe *Saccharomyces boulardii,* und
- mindestens eine andere Substanz, die zur Vorbeugung oder Behandlung affektiver Störungen bestimmt ist,
gegebenenfalls in Verbindung mit mindestens einem pharmazeutisch verträglichen Träger oder Vehikel.

14. Produkte, enthaltend:
- Zellen der Hefe *Saccharomyces boulardii,* und
- mindestens eine andere Substanz, die zur Vorbeugung oder Behandlung affektiver Störungen bestimmt ist,
als Kombinationsprodukt zum getrennten, gleichzeitigen oder über einen Zeitraum verteilten Gebrauch zur Vorbeugung oder Behandlung affektiver Störungen, insbesondere in der Vorbeugung oder Behandlung depressiver Störungen oder von Depression oder von Angststörungen oder Angst, oder zum Gebrauch als Antidepressivum oder Anxiolytikum.

## Claims

1. *Saccharomyces boulardii* yeast cells for use in the prevention or treatment of mood disorders in an individual.

2. *Saccharomyces boulardii* yeast cells for use according to claim 1, in the prevention or treatment of depressive disorders, in particular selected from the group consisting of substance/drug-induced depressive disorder, disruptive disorder with mood dysregulation, major depression, persistent depressive disorder, depressive disorder induced by another medical condition, and any depressive disorder specified and unspecified, or depression, or for use as an antidepressant, in an individual.

3. *Saccharomyces boulardii* yeast cells for use according to claim 1 or 2, in the prevention or treatment of anxiety disorders or anxiety, in particular selected from the group consisting of separation anxiety, selective mutism, specific phobia, social phobia, panic disorder, agoraphobia, generalized anxiety disorder, drug/substance anxiety disorder, anxiety disorder caused by another medical condition, and any anxiety disorder specified and unspecified, or for use as an anxiolytic, in an individual.

4. *Saccharomyces boulardii* yeast cells for use according to any one of claims 1 to 3, the *Saccharomyces boulardii* yeast cells being lyophilized.

5. *Saccharomyces boulardii* yeast cells for use according to any one of claims 1 to 4, the *Saccharomyces boulardii* yeast cells being in a form suitable for oral administration, in particular in the form of capsules or sachets of powder.

6. *Saccharomyces boulardii* yeast cells for use according to any one of claims 1 to 5, the yeast cells being administered at a dose of 0.00125 to 25 g/kg/d.

7. *Saccharomyces boulardii* yeast cells for use according to any one of claims 1 to 6, in combination with at least one other substance intended for the prevention or treatment of mood disorders.

8. Pharmaceutical composition or medicament comprising *Saccharomyces boulardii* yeast cells as active principle and optionally at least one pharmaceutically acceptable vehicle or excipient, for use in the prevention or treatment of mood disorders, in particular depressive disorders or depression, in particular selected from the group consisting of substance/drug-induced depressive disorder, disruptive disorder with mood dysregulation, major depression, persistent depressive disorder, depressive disorder induced by another medical condition, and any specified and unspecified depressive disorder, or anxiety disorders or anxiety, in particular selected from the group consisting of separation anxiety, selective mutism, specific phobia, social phobia, panic disorder, agoraphobia, generalized anxiety disorder, substance/drug-induced anxiety disorder, and anxiety disorder induced by another medical condition.

9. A pharmaceutical composition or medicament for use according to claim 8, wherein the *Saccharomyces boulardii* yeast cells are lyophilized.

10. Pharmaceutical composition or medicament for use according to claim 8 or 9, in a form suitable for oral administration, in particular in the form of capsules or sachets of powder.

11. Pharmaceutical composition or medicament for use according to any one of claims 8 to 10, comprising the *Saccharomyces boulardii* yeast cells in a unit dose of 50 to 250 mg.

12. Pharmaceutical composition or medicament for use according to any one of claims 8 to 11, comprising at least one other substance intended for the prevention or treatment of mood disorders.

13. Pharmaceutical composition or medicament comprising as active substance:
- *Saccharomyces boulardii* yeast cells, and
- at least one other substance intended for the prevention or treatment of mood disorders,
optionally in combination with at least one pharmaceutically acceptable vehicle or excipient.

14. Products containing:
- *Saccharomyces boulardii* yeast cells, and
- at least one other substance intended for the prevention or treatment of mood disorders,
as a combination product for separate, simultaneous or sequential use for the prevention or treatment of mood disorders, in particular in the prevention or treatment of depressive disorders or depression, or anxiety disorder or anxiety, or for use as an antidepressant or anxiolytic.
